# EUROPEAN PATENT SPECIFICATION

(11) **EP 3 887 455 B1**
(45) Date of publication and mention of the grant of the patent: **27.12.2023**
(21) Application number: 19813948.7
(22) Date of filing: 27.11.2019
(51) Int. Cl.: C09D 5/00

(54) **COMPOSITIONS INCLUDING A LASER MARKING ADDITIVE AND SYSTEMS AND METHODS OF LASER MARKING THE COMPOSITIONS**
ZUSAMMENSETZUNGEN MIT EINEM LASERMARKIERUNGSADDITIV UND SYSTEME UND VERFAHREN ZUR LASERMARKIERUNG DER ZUSAMMENSETZUNGEN
COMPOSITIONS COMPRENANT UN ADDITIF DE MARQUAGE LASER ET SYSTÈMES ET PROCÉDÉS DE MARQUAGE LASER DES COMPOSITIONS

(30) Priority: 30.11.2018 EP 18209603
(43) Date of publication of application: 06.10.2021
(73) Proprietor: SHPP Global Technologies B.V., 4612 PX Bergen op Zoom (NL)
(72) Inventor: DE BROUWER, Johannes, 4612 PX Bergen op Zoom (NL); NANDI, Manish, 4612 PX Bergen op Zoom (NL); GUNBAS, Duygu Deniz, 4612 PX Bergen op Zoom (NL)
(74) Representative: Balder IP Law, S.L.
(86) International application number: PCT/IB2019/060246
(87) International publication number: WO 2020/110039

(56) References cited:
- EP-A1- 2 955 029
- WO-A2-2011/005631
- DE-A1- 10 228 186
- DE-A1- 19 848 555

## Description

### CROSS-REFERENCE TO RELATED APPLICATIONS

This application claims the benefit of priority of European Patent Application No. 18209603.2 filed November 30, 2018, which is hereby incorporated by reference in its entirety.

### FIELD OF INVENTION

The present disclosure relates generally, but is not limited to, to compositions for laser marking, system and methods of making the compositions, and systems and methods of laser marking the compositions.

### BACKGROUND

Unique Device Identification regulation requires producers of certain classes of medical devices to mark such devices with a unique identification (ID). For example, a product may require "direct marking", such as a marking that cannot be tampered with and that remains visible and discernable over the lifetime of the product. Over the course of the product's lifetime, the product (e.g., a medical device product) may be subjected to multiple cleaning and sterilization operations. Such cleaning and sterilization operations can remove a top layer of a product and markings thereon. When using ink/printing for the marking, the ink needs to be carefully adjusted to the material and/or surface preparation techniques are utilized to ensure good wettability. Surface preparation techniques may be required on low surface energy materials, such as plastics and in particular cyclic olefin copolymers (COCs), cyclic olefin polymers (COPs), cyclic block copolymers (CBC), pentaerythritol tetrastearate (PETs), and polyolefins. Even with proper ink section and surface preparation, ink adherence may not be permanent. For example, the ink of ink based markings can fade and wear away from repeated cleaning and sterilization operations which medical devices are subjected to. Thus, permanent direct marking of components, especially plastics, faces challenges of identifying one or more techniques and/or one or more materials that do not suffer from wear or fading and that are resistant to tampering.

EP2955029 A1 describes laser markable materials for IR laser marking. The laser markable materials include multiple layers, such as a laser markable layer with IR absorbing additives (pigments or dyes) and an outer layer (UV protective layer). EP2955029 A1 discloses using a single UV absorber as a UV protective additive which is preferably used in the outer layer of a multiple layer product for UV protection of an interior laser markable layer.

WO2011/005631 A2 describes potassium cesium tungsten bronze particles for IR laser marking. WO2011/005631 A2 discloses that the described tungsten bronze particles are suitable near infrared (NIR) absorbers and heat shielding additives. WO2011/005631 A2 discloses UV absorbers and light stabilizers as light protection additives for infrared laser marking.

DE10228186 A1 describes UV stabilized particles suitable as light protection additives. The UV stabilized particles described in DE10228186 A1 may be used as light protection for laser marking, i.e., protecting a conventional laser marking from UV light degradation.

DE19848555 A1 describes a perylene dye which can absorb UV light. DE19848555 A1 discloses that the perylene fluorescent dye can be used in laser printers which use a NIR laser and electrostatic etching to create an image.

### SUMMARY

The present disclosure describes a composition comprising one or more polymers; and two or more ultraviolet absorbing laser marking additives, and methods, devices, and systems to generate laser markings on substrates formed from the composition. The ultraviolet absorbing laser marking additive is selected from the group consisting of 2-(4,6-Diphenyl-1,3,5-triazin-2-yl- 5-hexyloxy)phenol, 2-(2h-benzotriazol-2-yl)-4-(1,1,3,3-tetramethylbutyl)phenol, 2-(2 hydroxy - 3,5 dicumyl)benzotriazole, and any combination thereof. Such ultraviolet absorbing laser marking additives described herein may be Food And Drug Administration (FDA) of the United States of America approved and/or European Food Safety Authority (EFSA) approved as "Food Safe" or "Food Grade" for "Food Contact Materials" or "Food Contact Substances". As described herein, ultraviolet (UV) refers to electromagnetic radiation having a wavelength of 10 nanometers (nm) to 400 nm.

The UV absorbing laser marking additive can be added to a transparent/translucent material and/or a low surface energy materials to enable direct marking by a light source (e.g., a UV light source), such as a UV laser. Illustrative, non-limiting examples of the transparent/translucent material and/or the low surface energy material include polycarbonate (PC), polymethyl methacrylate (PMMA), cyclic olefin copolymers (COCs), cyclic olefin polymers (COPs), cyclic block copolymers (CBC), pentaerythritol tetrastearate (PET), polyvinyl chloride (PVC), polyethylene (PE), polypropylene (PP), polystyrene (PS), polymetylpentene (PMP), isosorbide based polycarbonate, styrene acrylonitrile (SAN), acrylonitrile butadiene styrene (ABS), polylactic acid bisphenol-A based polycarbonate copolymers. To illustrate, polymer compositions including the UV absorbing laser marking additive has increased UV absorption, as compared to the polymer itself or the polymer composition, and enables UV laser marking of a transparent or translucent polymer. For example, exposure to UV wavelengths forms white or light-colored marks in the polymer material that have high transparency, high resistance of scratches, provided barrier to atmospheric constituents, and have a high resistance to breakage. Because, the laser mark can be formed by changing the polymer materials reflectivity, as opposed to a marking with ink, the laser mark can be as durable as the polymer material itself. To illustrate, to remove the laser mark a force sufficient to remove or damage the polymer material would need to be applied. These properties enable one or more products formed from the compositions (e.g., polymer compositions) to support permanent direct marking by UV laser. Additionally, the one or more UV absorbing laser marking additives described herein can be used in plastic materials at concentrations that satisfy FDA and/or EFSA food contact status regulations and that produce visible markings after UV exposure. Furthermore, such polymer compositions can be used to manufacture and mark plastic parts and containers using a single layer approach (i.e., having a single layer of a polymer composition) and having a combination of properties, which includes high transparency, resistance of scratches, barrier to atmospheric constituents, and resistance to breakage.

Additionally, methods described herein can control inscription of transparent, opaque, and/or colored thermoplastic compositions having low visible reflectivity with customizable, and, optionally, machine-readable light colored text, logos, barcodes, and images using a UV laser beam. The methods (and compositions) described herein enable light colored text, a logo, and/or another identifier to be inscribed or marked on or close to the surface of a thermoplastic composition (e.g., a transparent thermoplastic composition) at wavelengths of about 10 nm to 500 nm, specifically, 300 nm to 400 nm. To illustrate, a light colored mark can be inscribed on a transparent material. As described herein, light colored can mean a white mark, where white can generally be described as white, off white, bright white ivory, snow, pearl, antique white, chalk, milk white, lily, smoke, seashell, old lace, cream, linen, ghost white, beige, cornsilk, alabaster, paper, whitewash, etc.

The UV absorbers may be combined with (e.g., blended with) polymers, such as in a melt-compounding operation, to produce polymer compositions and/or products made therefrom with a desired transparency or translucency. Additionally, or alternatively, additives may be combined with (e.g., blended with) the polymer compositions to change a color or appearance of the laser marking. For example, a colorant can be added to a natural, uncolored material (e.g., neat resin) without changing the reflectivity. As described herein, a process or method has been developed to generate a light colored mark on transparent thermoplastic compositions, including compositions comprising polymers such as polycarbonate, bisphenol-A polycarbonate based copolymers, polyesters, polymethyl methacrylate (PMMA), polystyrene, polybutylene terephthalate, polyolefins, polyamides, polyvinylchloride, polylactic acid, and combinations comprising at least one of the foregoing, at or below the surface of an article, using laser beams having a wavelength less than or equal to 500 nm. Some polymers can produce such an effect but the method described herein can provide a method to generate compositions that can enhance the process of generating a laser mark or even make it possible to generate a laser mark in compositions where a laser beam cannot typically generate a mark.

The polymer composition (including the UV absorbers) may advantageously enable direct marking of transparent and translucent materials by UV laser marking. Examples of products that can be benefited by such laser markings with increased wear resistance and reduced breakage is medical devices and food containers. Accordingly, the present disclosure overcomes the existing challenges of forming permanent direct markings in low surface energy materials, such as plastics.

The present compositions comprises: one or more polymers; and two or more ultraviolet absorbing laser marking additive selected from the group consisting of: 2-(4,6-Diphenyl-1,3,5-triazin-2-yl- 5-hexyloxy)phenol, 2-(2h-benzotriazol-2-yl)-4-(1,1,3,3-tetramethylbutyl)phenol, 2-(2 hydroxy - 3,5 dicumyl)benzotriazole, and any combination thereof, wherein a weight percentage concentration of 2-(4,6-Diphenyl-1,3,5-triazin-2-yl- 5-hexyloxy)phenol is within a range of 0.01% to 0.5 % by weight of the composition.

Additionally, the present composition comprises a weight percentage concentration of 2-(2h-benzotriazol-2-yl)-4-(1,1,3,3-tetramethylbutyl)phenol within a range of 0.01% to 0.5 % by weight of the composition. Additionally, the present composition comprises a weight percentage concentration of 2-(2 hydroxy - 3,5 dicumyl)benzotriazole within a range of 0.01% to 3 % by weight of the composition.

In some of the foregoing embodiments of the composition, at least one polymer of the one or more polymers is selected from the group consisting of: polycarbonate (PC), polymethyl methacrylate (PMMA), cyclic olefin copolymers (COCs), cyclic olefin polymers (COPs), cyclic block copolymers (CBC), pentaerythritol tetrastearate (PET), polyvinyl chloride (PVC), polyethylene (PE), polypropylene (PP), polystyrene (PS), polymetylpentene (PMP), isosorbide based polycarbonate, styrene acrylonitrile (SAN), acrylonitrile butadiene styrene (ABS), and any combination thereof.

In some of the foregoing embodiments of the composition, at least one polymer of the one or more polymers is selected from the group consisting of: cyclic olefin copolymers (COCs), cyclic olefin polymers (COPs), cyclic block copolymers (CBC), polymetylpentene (PMP), polyolefins, and any combination thereof. In some of the foregoing embodiments of the composition, the one or more polymers include polycarbonate and 2-(2 hydroxy - 3,5 dicumyl)benzotriazole is present in an amount less than or equal to 3 % by weight of the composition. In some of the foregoing embodiments, the one or more polymers include pentaerythritol tetrastearate (PET) and 2-(2 hydroxy - 3,5 dicumyl)benzotriazole is present in an amount less than or equal to 0.5 % by weight of the composition.

The present disclosure describes also a part including a substrate formed from the some of the compositions of the foregoing embodiments, wherein the substrate is transparent or translucent, wherein the ultraviolet absorbing laser marking additive enables formation of a laser marking responsive to receiving UV light from a UV laser, and wherein the laser marking formed in the substrate by a UV laser is light-colored or white. Additionally, the part may be a medical device or may be incorporated into a medical device.

The present method of inscribing a substrate comprises: at the substrate comprising one or more polymers and two or more ultraviolet absorbing laser marking additive selected from the group consisting of: 2-(4,6-Diphenyl-1,3,5-triazin-2-yl- 5-hexyloxy)phenol, 2-(2h-benzotriazol-2-yl)-4-(1,1,3,3-tetramethylbutyl)phenol, 2-(2 hydroxy - 3,5 dicumyl)benzotriazole, and any combination thereof, wherein a weight percentage concentration of 2-(4,6-Diphenyl-1,3,5-triazin-2-yl- 5-hexyloxy)phenol is within a range of 0.01% to 0.5 % by weight of the composition, wherein a weight percentage concentration of 2-(2h-benzotriazol-2-yl)-4-(1,1,3,3-tetramethylbutyl)phenol is within a range of 0.01% to 0.5 % by weight of the composition, and wherein a weight percentage concentration of 2-(2-hydroxy-3,5 dicumyl)benzotriazole is within a range of 0.01% to 3% by weight of the composition; performing, initiating application of a laser beam to the substrate, where the laser beam has a wavelength of 500 nm or less; and ceasing the application of the laser beam to the substrate, where the application of the laser beam generates a laser marking on the substrate; wherein the ultraviolet absorbing laser marking additive enables formation of the laser marking. In some implementations, the method further comprises initiating movement of a laser device such that the laser beam moves in a pattern that corresponds to a laser inscription.

In some of the foregoing embodiments of the method, the laser beam has a wavelength of 400 nm or less, and the laser beam passes through one or more layers prior to impinging on the substrate. In some of the foregoing embodiments, a laser device that generates the laser beam has one or more operating characteristics selected from the group of operating characteristics consisting of: a power output between 1 and 3 Watts (W), a frequency between 10 and 50 kilohertz (kHz), a movement speed between 200 and 1600 millimeters per second (mm/s), and a power setting of 95%. In some implementations, a part includes a substrate having a laser marking formed by some of the foregoing embodiments of the present methods.

As used herein, various terminology is for the purpose of describing particular implementations only and is not intended to be limiting of implementations. For example, as used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed implementation, the term "substantially" may be substituted with "within [a percentage] of" what is specified, where the percentage includes .1, 1, or 5 percent; and the term "approximately" may be substituted with "within 10 percent of" what is specified. The phrase "and/or" means and or. To illustrate, A, B, and/or C includes: A alone, B alone, C alone, a combination of A and B, a combination of A and C, a combination ofB and C, or a combination of A, B, and C. In other words, "and/or" operates as an inclusive or.

The terms "comprise" (and any form of comprise, such as "comprises" and "comprising"), "have" (and any form of have, such as "has" and "having"), and "include" (and any form of include, such as "includes" and "including"). As a result, an apparatus that "comprises," "has," or "includes" one or more elements possesses those one or more elements, but is not limited to possessing only those one or more elements. Likewise, a method that "comprises," "has," or "includes" one or more steps possesses those one or more steps, but is not limited to possessing only those one or more steps.

Any implementation of any of the systems, methods, and article of manufacture can consist of or consist essentially of - rather than comprise/have/include - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb. Additionally, the term "wherein" may be used interchangeably with "where."

Further, a device or system that is configured in a certain way is configured in at least that way, but it can also be configured in other ways than those specifically described. The feature or features of one implementation may be applied to other implementations, even though not described or illustrated, unless expressly prohibited by this disclosure or the nature of the implementations.

Some details associated with the implementations are described above, and others are described below. Other implementations, advantages, and features of the present disclosure will become apparent after review of the entire application, including the following sections: Brief Description of the Drawings, Detailed Description, and the Claims.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures are drawn to scale (unless otherwise noted), meaning the sizes of the depicted elements are accurate relative to each other for at least the implementation depicted in the figures. Views identified as schematics are not drawn to scale.
**FIG. 1** is a diagram that illustrates an example of a system for laser inscribing using a polymer composition including a UV absorbing laser marking additive.
**FIGS. 2A, 2B, and 2C** each illustrate a scanned image of test results of laser inscribing a polymer composition including one or more UV absorbing laser marking additives of FIG. 1 on a black background.
**FIGS. 3A, 3B, and 3C** each include a graph corresponding to intensity values of the test results of FIGS. 2A-2C.
**FIG. 4** is a perspective view of an example of a system for producing a part.
**FIG. 5** is a flowchart illustrating an example of a method of inscribing a substrate.
**FIG. 6** is a flowchart illustrating an example of a method of manufacturing a polymer composition including a laser marking additive.
**FIG. 7** is a flowchart illustrating an example of a method of manufacturing a part using a polymer composition including a laser marking additive.

### DETAILED DESCRIPTION OF ILLUSTRATIVE IMPLEMENTATIONS

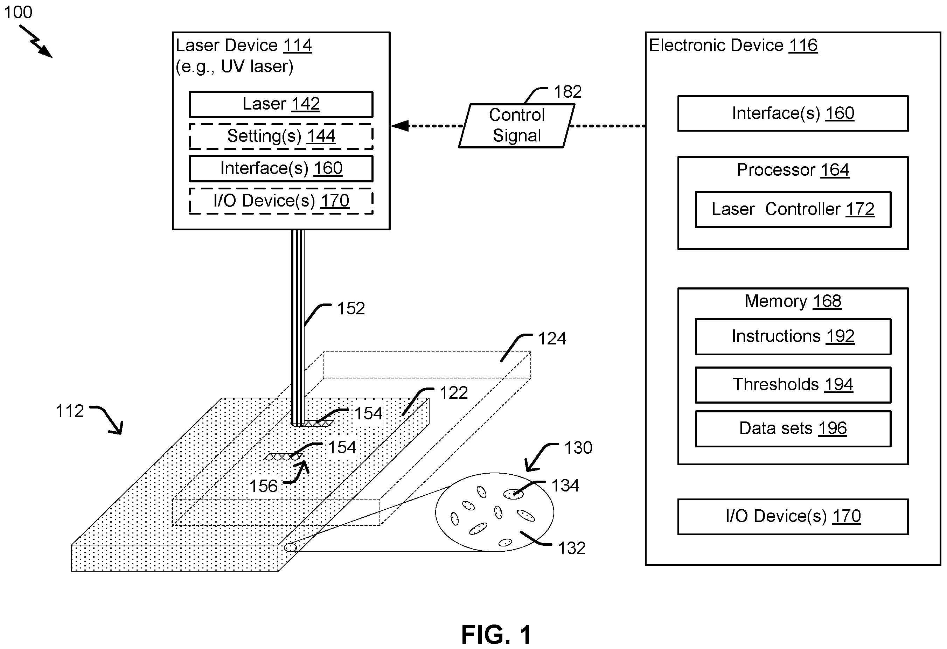

Referring to FIG. 1, a block diagram of a system 100 for inscribing a part 112, such as a molded part, is shown. Part 112 is formed from a polymer composition having a laser marking additive 134. Part 112 may be translucent or transparent and may be configured to enable generation or formation of laser markings 154 when exposed to UV radiation. For example, laser marking additive 134 includes an FDA or EU approved UV absorber and enables formation of white or light colored laser markings which may be associate with laser inscription and/or part identification.

System 100 includes part 112, a laser device 114, and an electronic device 116. Part 112 includes a substrate layer 122 (e.g., a first layer) and, optionally, an outer layer 124 (e.g., a second layer) coupled to substrate layer 122. Outer layer 124 is illustrated as extending past substrate layer 122 for illustrative purposes. Outer layer 124 may cover all or only a portion of substrate layer 122 and optionally cover all or a portion of another layer. In other implementations, part 112 may include one or more other layers, such as additional outer layers 124, intermediary layers, or base layers. In some such implementations, substrate layer 122 may be coupled to one or more of the other layers via the outer layer 124 or directly, such as by surface contact of substrate layer 122 and the one or more of the other layers.

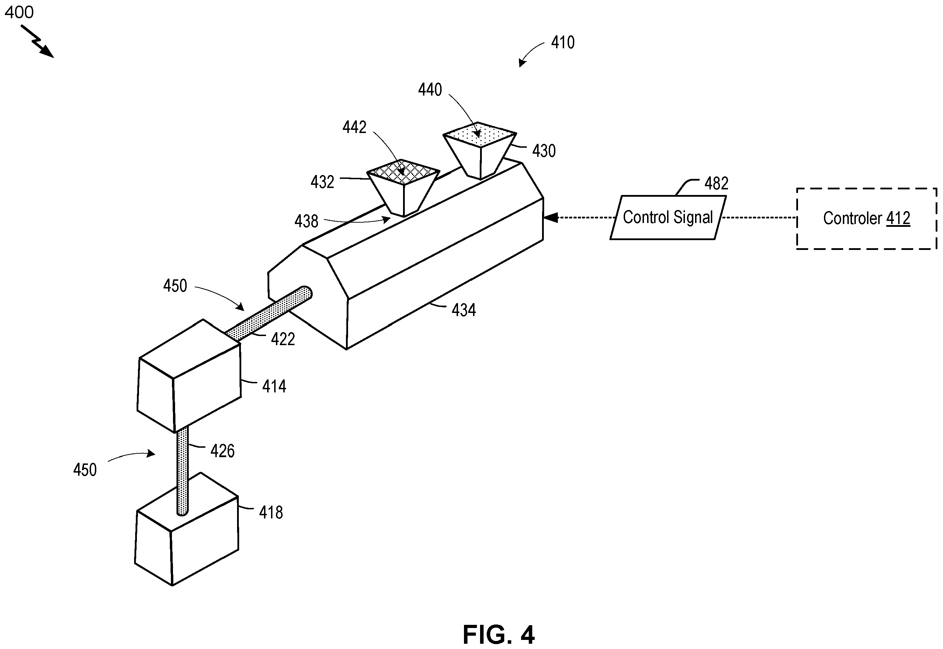

As illustrated in detailed view 130 of substrate layer 122, substrate layer 122 includes (i.e., is formed of) one or more polymers 132 and a laser marking additive 134. For example, substrate layer 122 is formed from a polymer composition, such as polymer composite 450 as described with reference to FIG. 4, including one or more polymers 132 and laser marking additive 134. Formation of the polymer composition is described with reference to FIG. 4. Optionally, substrate layer 122 further includes other additives, such as other laser marking additives (e.g., carbon black), other ultraviolet absorbers, or polymer additives, as described further with reference to FIG. 4.

Polymer 132 may include or correspond to a polymer or polymer blend, and is optionally transparent or translucent. In some implementations, polymer 132 includes polycarbonate (PC), polymethylmethacrylate (PMMA), cyclic olefin copolymers (COCs), cyclic olefin polymers (COPs), cyclic block copolymers (CBC), pentaerythritol tetrastearate (PET), polyvinyl chloride (PVC), polyethylene (PE), polypropylene (PP), polystyrene (PS), polymetylpentene (PMP) (e.g., TPX, a trademark of Mitsui Chemicals, Inc.), isosorbide based polycarbonate (e.g., Durabio, a trademark of Mitsubishi Chemical Corp.), styrene acrylonitrile (SAN), acrylonitrile butadiene styrene (ABS), or a combination thereof. In a particular implementation, polymer 132 is selected from the group consisting of cyclic olefin copolymers (COCs), cyclic olefin polymers (COPs), cyclic block copolymers (CBC), polymetylpentene (PMP), and polyolefins. Additional polymers are described with reference to FIG. 4.

Laser marking additive 134 is configured to interact with electromagnetic radiation (e.g., a laser beam 152) to form a laser marking 154 in or on substrate layer 122. Multiple laser markings 154 may be generated in substrate layer 122 to form a laser indicia 156 (e.g., a laser inscription), such as a pattern of laser markings 154. Exemplary laser indicia 156 include human readable indicia (such as text and/or numbers), machine readable indicia (such as bar codes, QR codes, patterns of dots, dashes, and or symbols, etc.), or a combination thereof.

In some implementations, laser marking additive 134 includes or corresponds to an ultraviolet absorber (UV absorber). A UV absorber is a molecule used in organic or synthetic materials to absorb UV radiation. The UV absorbers are configured to absorb at least a portion of UV radiation of the UV spectrum. For example, UVA absorbers are configured to absorb UVA radiation, i.e., electromagnetic radiation having wavelengths between 300 and 400 nm.

In some implementations, the UV absorber (of additive 134) includes 2-(4,6-Diphenyl-1,3,5-triazin-2-yl- 5-hexyloxy)phenol, 2-(2h-benzotriazol-2-yl)-4-(1,1,3,3-tetramethylbutyl)phenol, 2-(2 hydroxy - 3,5 dicumyl)benzotriazole, or a combination thereof. In other implementations, the UV absorber includes one or more other FDA or EU approved food safe UV absorbers.

A total concentration of one or more UV absorbers is between 0.01 and 10 wt% of the substrate layer 122 (e.g., wt% of a polymer composition used to form substrate layer 122 excluding fillers). For example, a concentration of a first UV absorber is 2 wt%, a concentration of a second UV absorber is 4 wt%, and a concentration of a third UV absorber is 3 wt%. In some implementations, a concentration of a particular UV absorber is between 0.01 and 6 wt%. In a particular implementation, a concentration of a particular UV absorber is between 0.1 and 3 wt%.

Concentrations of a particular UV absorber may depend on the polymer 132 of the substrate layer 122. For example, a concentration of 2-(2 hydroxy - 3,5 dicumyl)benzotriazole of the substrate layer 122 is less than or equal to 0.5 wt% when polymer 132 is or includes pentaerythritol tetrastearate (PET) and is less than or equal to 3 wt% when polymer 132 is or includes another polymer, such as polycarbonate (PC). To illustrate, when polymer 132 is or includes a concentration of PET of greater than or equal to 50 wt% of the substrate layer 122, a concentration of 2-(2 hydroxy - 3,5 dicumyl)benzotriazole is 0.5 wt%.

An illustrative exemplary combination of multiple UV absorbers includes 0.5 wt% of 2-(4,6-Diphenyl-1,3,5-triazin-2-yl- 5-hexyloxy)phenol and at least one of 0.5 wt% of 2-(2h-benzotriazol-2-yl)-4-(1,1,3,3-tetramethylbutyl)phenol or 3.0 wt% of 2-(2 hydroxy - 3,5 dicumyl)benzotriazole, in a polycarbonate based polymer. Another illustrative exemplary combination of multiple UV absorbers includes 0.5 wt% of 2-(2h-benzotriazol-2-yl)-4-(1,1,3,3-tetramethylbutyl)phenol and at least one of 0.5 wt% of 2-(4,6-Diphenyl-1,3,5-triazin-2-yl- 5-hexyloxy)phenol or 3.0 wt% of 2-(2 hydroxy - 3,5 dicumyl)benzotriazole, in a polycarbonate based polymer.

In some implementations, part 112 includes outer layer 124 (e.g., an exterior layer) coupled to substrate layer 122. Outer layer 124 may include or correspond to a cap layer or cover film of substrate layer and may protect laser markings 154 of the substrate layer 122 from wear and/or damage. In some implementations, outer layer 124 is transparent or translucent to UV radiation. For example, outer layer 124 enables UV radiation to pass through outer layer 124 to substrate layer 122, such that outer layer 124 does not scatter the UV radiation. Outer layer 124 may have a low refractive index to UV radiation, such as a refractive index of 1 to 2 and may be free of (i.e., not include) UV absorbers.

Laser device 114 is configured to generate laser indicia 156 in part 112, such as substrate layer 122 thereof. Laser device 114 includes laser 142 and optionally includes one or more interfaces 160 and/or one or more input/output (I/O) devices 170. Laser 142 is configured to generate a laser beam 152 and to direct or provide the laser beam 152 to part 112 to form laser markings 154. By moving laser 142 (and thus laser beam 152) multiple laser markings 154 can be formed to generate laser indicia 156. In some implementations, one or more interfaces 160 and/or one or more I/O devices 170 are configured to adjust settings or parameters of laser 142, i.e., laser settings. Laser settings include a power output of the laser 142, a power setting of the laser 142, a frequency of the laser 142, and a movement speed of the laser 142.

Laser 142 includes or corresponds to a gas laser, a laser diode, a solid-state laser, an excimer laser, or a combination thereof. In some implementations, laser 142 is a UV laser and is configured to generate laser beam 152 having electromagnetic radiation of UV wavelengths. For example, laser 142 is a UVA laser (315-400 nm), a UVB laser (280-315 nm), a UVC laser (100-280 nm), or an extreme UV laser (10-121 nm). In a particular implementation, laser 142 is a UVA laser and has a wavelength of substantially 355 nm. In other implementations, laser 142 has a wavelength of less than or equal to 500 nm. In some implementations, laser 142 operates at a power setting of substantially 95 percent. The power setting of laser 142 is a value of 1-100 percent and controls or adjusts an output power of the laser 142 and a strength or power of the laser beam 152. In some implementations, the laser 142 has power output of between 1 and 3 Watts (W). In a particular implementation, the laser 142 has a power output of substantially 2 W at a power setting of substantially 95 percent.

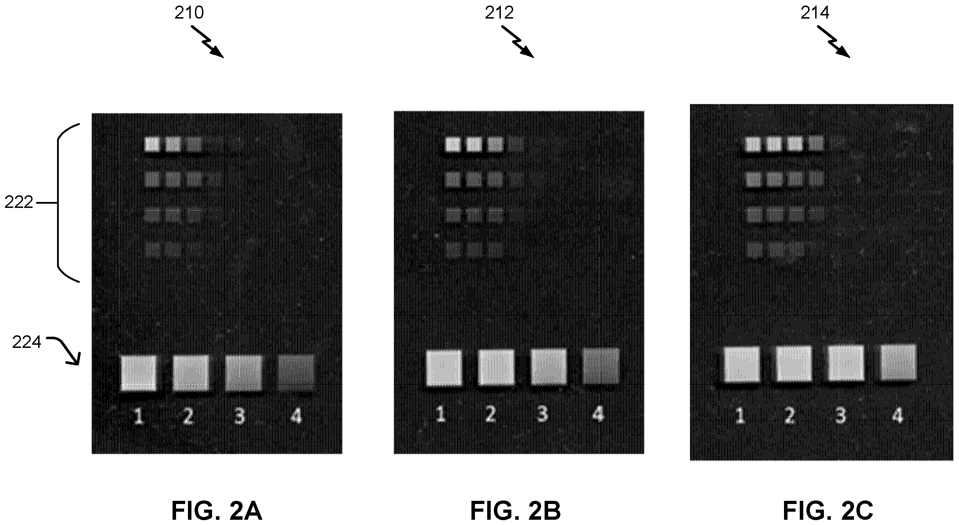

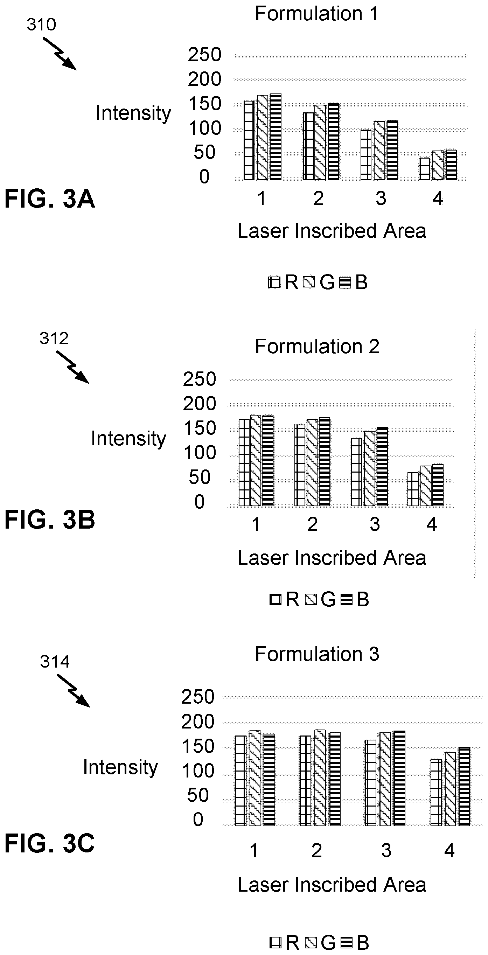

In some implementations, laser 142 has a frequency between 10 and 50 kilohertz (kHz). In a particular implementation, laser 142 has a frequency between 20 and 40 kHz. In other implementations, laser 142 has a frequency that is greater than 50 kHz. Alternatively, laser 142 has a frequency that is less than or equal to any one of or that is between any two of substantially: 10, 15, 20, 25, 30, 35, 40, 45, and 50 kHz. In some implementations, laser 142 has a movement speed between 200 to 1600 millimeters per second (mm/s). In a particular implementation, laser 142 has a movement speed between 600 to 1200 mm/s. In other implementations, laser 142 has a movement speed that is greater than 1600 mm/s. Alternatively, laser 142 has a movement speed that is less than or equal to any one of or that is between any two of substantially: 200, 300, 400, 500, 600, 700, 800, 900, 1000, 1100, 1200, 1300, 1400, 1500, and 1600 mm/s. Additionally detail regarding illustrative laser setting combinations are described further with reference to FIGS. 2A-2C and 3A-3C.

Electronic device 116 includes one or more interfaces 160, one or more processors (e.g., one or more controllers), such as a representative processor 164, a memory 168, and one or more input/output (I/O) devices 170. Interfaces 160 may include a network interface and/or a device interface configured to be communicatively coupled to one or more other devices, such as laser device 114. For example, interfaces 160 may include a transmitter, a receiver, or a combination thereof (e.g., a transceiver), and may enable wired communication, wireless communication, or a combination thereof. Although electronic device 116 is described as a single electronic device, in other implementations system 100 includes multiple electronic devices. In such implementations, such as a distributed control system, the multiple electronic devices each control a device or sub-system of system 100, such as laser device 114.

Processor 164 includes laser controller 172. For example, laser controller 172 (e.g., processor 164) may be configured to generate and/or communicate one or more laser setting control signals 182 to laser device 114 to initiate and control laser inscription of laser indicia 156. To illustrate, laser controller 172 sends a laser setting control signal 182 to laser device 114 to adjust a particular laser setting.

Although one or more components of processor 164 are described as being separate components, at in some implementations, one or more components of processor 164 may be separate from (e.g., not included in) processor 164. To illustrate, laser controller 172 may be separate and distinct from processor 164.

Memory 168, such as a non-transitory computer-readable storage medium, may include volatile memory devices (e.g., random access memory (RAM) devices), nonvolatile memory devices (e.g., read-only memory (ROM) devices, programmable read-only memory, and flash memory), or both. Memory 168 may be configured to store instructions 192, one or more thresholds 194, and one or more data sets 196. Instructions 192 (e.g., control logic) may be configured to, when executed by the one or more processors 164, cause the processor(s) 164 to perform operations as described further here. For example, the one or more processors 164 may perform operations as described with reference to FIGS. 4-7. The one or more thresholds 194 and one or more data sets 196 may be configured to cause the processor(s) 164 to generate control signals. For example, the processors 164 may generate and send control signals (e.g. 182) responsive to receiving sensor data, such as sensor data from laser device 114 or a monitoring device (not shown). The laser settings can be adjusted based on comparing sensor data to one or more thresholds 194, one or more data sets 196, or a combination thereof.

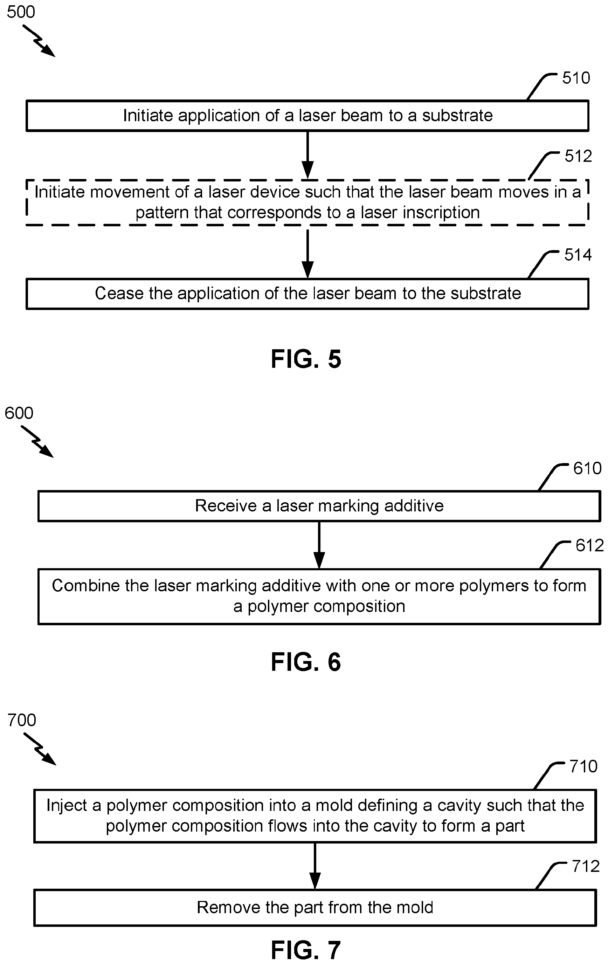

In some implementations, processor 164 may include or correspond to a microcontroller/microprocessor, a central processing unit (CPU), a field-programmable gate array (FPGA) device, an application-specific integrated circuits (ASIC), another hardware device, a firmware device, or any combination thereof. Processor 164 may be configured to execute instructions 192 to initiate or perform one or more operations described with reference to FIG. 4 and/or one more operations of the methods of FIGS. 5-7.

The one or more I/O devices 170 may include a mouse, a keyboard, a display device, the camera, other I/O devices, or a combination thereof. In some implementations, the processor(s) 164 generate and send control signals responsive to receiving one or more user inputs via the one or more I/O devices 170.

Electronic device 116 may include or correspond a communications device, a mobile phone, a cellular phone, a satellite phone, a computer, a tablet, a portable computer, a server, a display device, a media player, or a desktop computer. Additionally, or alternatively, the electronic device 116 may include a set top box, an entertainment unit, a personal digital assistant (PDA), a monitor, a computer monitor, a television, a tuner, a video player, any other device that includes a processor or that stores or retrieves data or computer instructions, or a combination thereof.

During operation of system 100, laser 142 of laser device 114 generates laser beam 152 and directs/provides laser beam 152 to substrate layer 122. For example, electronic device 116 sends a control signal 182 to laser device 114 to initiate generation of laser beam 152 by laser device 114. As another example, a user of laser device 114 generates laser beam 152 by providing one or more inputs to one or more I/O devices 170 of laser device 114.

In some implementations, laser beam 152 passes through one or more outer layers 124 prior to contacting or impinging on substrate layer 122. In a particular implementation, the substrate layer 122 is transparent or translucent. For example, substrate layer 122 (e.g., polymer 132 thereof) is transparent or translucent to visible light and UV light (e.g., UV radiation). To illustrate, substrate layer 122 (e.g., polymer 132 thereof) has a refractive index of substantially 1 to 2.

Laser marking additives 134 in substrate layer 122 react to from laser marking responsive to laser exposure of laser beam 152. For example, UV absorber type laser marking additives 134 absorb UV radiation of laser beam 152 and cause the substrate layer 122 to deform to form laser marking 154. To illustrate, absorption of UV radiation causes UV absorbers to increase in temperature, which increases a temperature of an area of the substrate layer 122 surrounding the UV absorbers. The localized increase in temperature of the substrate layer 122 causes the substrate layer 122 (e.g., polymer 132 thereof) to deform (e.g., plastically or thermally deform) to form laser marking 154, such as a white or light colored marking. Exemplary laser markings are shown in FIGS. 2A-2C.

In some implementations, laser device 114 moves or directs laser 142 to move laser beam 152 to generate multiple laser markings 154 to form laser indicia 156 (e.g., laser inscription or laser ID). For example, electronic device 116 sends one or more control signals 182 to adjust laser settings of the laser 142, to move laser 142, and/or to cease generation of laser beam 152. The laser markings 154 may be formed on a surface of substrate layer 122 (e.g., a surface coupled to or in contact with outer layer 124) or within the substrate layer 122.

In some implementations, part 112 is included in a medical device, or includes or corresponds to a medical device. Laser indicia 156 of part 112 enable a unique identifier to be directly marked on part 112, as opposed to being placed on a removable label or placard. In such implementations where part 112 is a medical device, laser indicia 156 enables tracking of the medical device and is resistant to medical cleaning procedures and/or tampering.

Thus, system 100 of FIG. 1 enables generation of laser markings in transparent or translucent polymers with UV lasers. Such laser markings can be used to inscribe or directly mark medical devices to identify the medical devices and comport with regulatory direct marking standards. Additionally, such laser markings can be made in polymers having concentrations of UV absorbers which comport with FDA or EFSA regulations regarding food contact safety. Accordingly, the present disclosure overcomes the existing challenges of forming wear resistant laser markings in transparent or translucent polymers with UV lasers. The laser markings enable compliance with regulatory standards and prevent wear and/or tampering of the laser markings.

In some implementations, exposure from the UV laser generates microdots in substrate layer 122 (e.g., polymer 132 thereof) of different optical properties (e.g., reflectivity) as compared to polymer 132 or a background material such that the laser mark can be clearly visible (e.g., identifiable by the human eye without magnification) to semi-covert (e.g., barely visible to the human eye). For example, UV exposure can produce a thermal interaction in the polymer material. In other words, laser marking 154 can be a result of localized melting in the substrate layer 122 (e.g., polymer 132 thereof) to generate an extremely small optically different, reflective or light scattering dot or array of dots. As compared to other lasers (e.g., infrared lasers) which form carbonized marks from surface modification and increase a void volume of the material, laser markings 154 disclosed herein can form as a result of near surface modification. Thus, system 100 and the methods disclosed herein can generate laser marking 154 without increasing the void volume of the material.

Additionally, the intensity and color of laser marking 154 can be modulated by varying laser parameters such as power, frequency, speed, line spacing, and focus, which allows marks of varying degrees of legibility or clarity to be generated. The legibility of the laser marking 154, whether on or close to the surface of substrate layer 122, can be modulated from an easily readable light colored mark to a semi-covert, barely visible markings, which may require a particular viewing device, such as a specialized and sophisticated viewing device, to read and interpret the data. The resulting watermark ("ghost mark") can be used as a semi-covert method of identification and/or traceability for material or product identification. A watermark can generally be described as a recognizable image that appears when viewed by transmitted light (or when viewed by reflected light, atop a dark background) at specific angles. Watermarks can vary greatly in their visibility; while some are obvious on casual inspection, others require some study to observe.

In some implementations, such as one layer parts formed from one shot processes, the laser marking 154 can be located on the surface of the first component and/or the substrate. In other processes, such as two layer parts formed from two shot molding, over-molding, or a laser welded set-up, the laser marking 154 can be located within the substrate layer 122 (e.g., in the first shot) with no interaction with a base material or other layer 124. To illustrate, laser marking 154 occurs at the interface of two components or layers (e.g., 122, 124). For example, an outer layer 124 of PMMA does not interact with the laser beam 152 and a substrate layer 122 of PC including a UV laser marking additive 134 interacts with the laser beam 152 to alter the optical properties (e.g., reflectivity) of the substrate layer 122 at the point of interaction. In either case, the laser marking 154 can be durable (i.e., cannot be scratched off the surface).

In some implementations, the appearance of the laser marking 154 can change when viewed from either side of the substrate layer 122. For example, with a neat resin (i.e., no colorant), the laser marking 154 appears similar from both sides of the part 112 (i.e., front and back). The use of electromagnetic radiation to create a visibly perceivable laser marking 154 on a polymer material as described herein can range from clearly visible, poorly visible and/or visible under only specific lighting/viewing conditions (e.g., a watermark). The appearance of the laser marking 154 can be manipulated by controlling the change in morphology of the polymeric material by changing the electromagnetic radiation variables.

Various type of parts 112 can be inscribed. The following description of parts 112 is merely illustrative of parts 112 that can be produced and inscribed using system 100 (and/or the methods disclosed herein) and are not intended to limit the scope hereof. For example, the system 100 and the methods disclosed herein can be used to laser inscribe logos, texts, barcodes, and/or images (e.g., photographic images) in the following exemplified parts 112: medical devices, pharmaceutical or food packaging marks, including watermarks, glazing parts such as automotive panels and lamp bezels in which the mark, including a watermark, can be introduced on the surface of the part, pre- or post-coating and can also be placed over second shot areas to increase contrast; weapons (e.g., firearms); electronic housings or screens in phones, computers, tablets, televisions, etc., where the mark, including a watermark, is on the surface or at the interface of two layers or components.

In a particular example, a laser marking 154 can be generated on the surface or subsurface at the interface of two layers within cards or tickets, such as business cards, identification (ID) cards, customer cards, etc. The laser marking 154 can be generated even when the entire card is transparent or exists as a window in an opaque card. Other possible layers in the ID card can include a metallic layer, a magnetic layer, a layer with angular metamerism properties, and combinations thereof. The layers can be assembled via various processes including, but not limited to co-extrusion, co-lamination, etc.

More specifically, ID cards can include a core layer (e.g., reflective thermoplastic layer), and a transparent film layer comprising the compositions disclosed herein (e.g., a material comprising a UV absorbing additive having the capability of absorbing light at wavelengths less than or equal to 500 nm). Optionally, a cap layer (e.g., outer layer 124) can be disposed on an exterior of the transparent film layer opposite the core layer, e.g., to protect against scratches, provide added chemical resistance, and/or light resistance. Other layers having a thickness of less than or equal to 100 micrometers can be formed first by an extrusion, a melt casting, or solvent casting process, and optionally, stretched to reach the desired thickness. The cap layers, and other, optional layer(s) can be added in the form of a coating.

FIGS. 2A-2C and 3A-3C illustrate test results of laser inscription of polymer compositions including laser marking additives 134 of FIG. 1. Each of FIGS. 2A-2C correspond to a particular formulation of a polymer composition and depict a scanned image of multiple laser inscriptions made in the respective polymer composition using different laser settings on a black background. Each of FIGS. 3A-3C are a graph corresponding to intensity values of the laser inscriptions of FIGS. 2A-2C, respectively.

Referring to FIGS. 2A-2C, test pieces 210-214 of different compositions (e.g., Formulations 1-3) were formed according to the materials and concentrations (wt%) listed in Table 1.

| Table 1 | | | |
|---|---|---|---|
| | **Formulation 1** | **Formulation 2** | **Formulation 3** |
| PC 105 | 17.34 | 17.34 | 17.34 |
| PC 175 | 81.96 | 81.92 | 81.82 |
| 2-(2 hydroxy-5-t-octylphenyl)benzotriazole | 0.15 | 0.20 | 0.30 |
| Tris(2,4-di-t-butylphenyl)phosphite | 0.05 | 0.05 | 0.05 |
| Pentaerythritol Tetrastearate (PETS) | 0.27 | 0.27 | 0.27 |
| 0.1% Pig-Blu-60 in PC carrier | 0.1029 | 0.1029 | 0.1029 |
| 0.15% Sol-Vlt-36 in PC carrier | 0.122 | 0.122 | 0.122 |

FIG. 2A depicts laser inscriptions on a first test piece 210 formed from a polymer composition according to a first formulation (Formulation 1). Similarly, FIGS. 2B and 2C depict laser inscriptions on a second test piece 212 formed from a polymer composition according to a second formulation (Formulation 2) and laser inscriptions on a third test piece 214 formed from a polymer composition according to a third formulation (Formulation 3).

The test pieces 210-214 were laser inscribed with a 355 nm laser beam to evaluate the effect of different concentrations of UV absorbers. Specifically, a Trumark 6330 laser (355 nm), which had a power output of 2 Watts (W) was used to inscribe test pieces 210-214 using a range of laser settings, i.e., laser settings 1-4 (as described below). A power setting of the laser was set to 95% and a line spacing of 0.03 mm was used in all laser settings. The line spacing, also referred to as fill factor, denotes a spacing between adjacent lines and laser dots (spacing between beam widths directed at test pieces 210-214). Thus, the power setting, line spacing and power were kept constant in laser settings 1-4 and the frequency and the movement speed of the laser were varied in laser settings 1-4.

The Trumark 6330 laser was used to mark square areas 222 of test pieces 210-214 with varying settings. The test pieces 210-214 had a thickness of 2.54 mm. To measure an intensity contrast (e.g., lightness) of inscribed areas, larger 0.5 mm x 0.5 mm square areas, i.e., measured areas 224, were marked. In FIGS. 2A-2C, measured areas 224 (e.g., 4 larger bottom squares) were inscribed with the same laser settings as those used in the first line of marks of the square areas 222. Each measured area 224 was marked with a different laser settings, that is laser settings 1-4.

Laser setting 1 had a frequency of 20 kilohertz (kHz) and a laser speed of 600 millimeters per second (mm/s). Laser setting 2 had a frequency of 25 kHz and a laser speed of 750 mm/s. Laser setting 3 had a frequency of 30 kHz and a laser speed of 900 mm/s. Laser setting 4 had a frequency of 40 kHz and a laser speed of 1200 mm/s. Intensities of the measured areas 224 were measured according to the following mythology.

To obtain color intensities, the test pieces 210-214 were placed on a black background to emphasize contrast. A reference white and a reference black were used of RAL 9010 and RAL 9005 to measure the color intensities. The color intensities in RGB color space for the Reference white (RAL 9010) and the Reference black (RAL 9005) are shown in Table 2.

| Table 2 | | | |
|---|---|---|---|
| | Red Intensity Value | Green Intensity Value | Blue Intensity Value |
| Reference White (RAL9010) | 255 | 255 | 255 |
| Reference Black (RAL9005) | 0 | 0 | 0 |

The RGB intensities (e.g., intensity values or pixel values) correspond to 8-bit color values (0-255) with 0 indicating no color or intensity, and 255 indicating full color or intensity in a particular color plane (i.e., red, green, or blue). To perform the color intensity measurement of test pieces 210-214, the test pieces 210-214 were scanned alongside the Reference white and the Reference black. The scanned images were processed by Gimp 2.4 software to generate the RGB values. To illustrate, the Gimp 2.4 software determines, from each scanned image, an intensity value for each color plane (RGB) using the Reference white and black in each scanned image as reference values for zero intensity (0) and full intensity (255).

The results of the color intensity measurement of the measured areas 224 of test pieces 210-214 are listed in Table 3 and depicted graphically in FIGS. 3A-3C.

| Table 3 | | | | |
|---|---|---|---|---|
| Formulation | Laser Setting | Red Intensity Value | Green Intensity Value | Blue Intensity Value |
| 1 | 1 | 157 | 169 | 171 |
| | 2 | 135 | 149 | 153 |
| | 3 | 101 | 115 | 118 |
| | 4 | 43 | 57 | 58 |
| 2 | 1 | 170 | 180 | 178 |
| | 2 | 161 | 172 | 175 |
| | 3 | 135 | 147 | 156 |
| | 4 | 65 | 79 | 82 |
| 3 | 1 | 174 | 183 | 178 |
| | 2 | 174 | 185 | 182 |
| | 3 | 168 | 180 | 184 |
| | 4 | 129 | 143 | 152 |

FIGS. 3A-3C illustrate bar graphs of the RGB intensity values of Table 3, i.e., the RGB intensity values of the laser markings generated by the four different laser settings 1-4 on test pieces 210-214 shown in FIGS. 2A-2C. In FIGS. 3A-3C, the horizontal axis depicts laser settings 1-4 and the vertical axis illustrates intensity values for each laser setting. Each laser settings has three bars representing red, green and blue intensity values.

Referring to FIG. 3A, a graph 310 illustrates intensity values for laser markings on Formulation 1 using laser settings 1-4 as shown in FIG. 2A. In FIG. 3A, laser intensity values for each color are similar, but laser intensity value decrease from laser setting 1 to laser setting 4, i.e., laser marking intensity decreases as the frequency and movement speed are both increased.

FIG. 3B depicts graph 312 illustrating intensity values for laser markings on Formulation 2 using laser settings 1-4 as shown in FIG. 2B, and FIG. 3C depicts graph 314 illustrating intensity values for laser markings on Formulation 3 using laser settings 1-4 as shown in FIG. 2C. FIGS. 3B and 3C depict similar trends. However, in FIG. 3C, the decrease in intensity value from laser setting 1 to laser setting 4 is much less, that is the increase in frequency and movement speed does not affect that intensity value to the same extent as in FIGS 3A and 3B. Additionally, Formulation 3 results in the highest RGB intensities for all four laser settings used. Higher intensities correspond to a whiter or lighter mark. Thus, the laser marks achieved in Formulation 3 are the lightest among these three formulations.

Thus, Formulation 3 enables a wider laser marking processing window (i.e., a wider range and combination of laser settings) as indicated by the lighter marks (higher RGB intensities) obtained with the same laser marking parameters (laser setting 4), as compared to the laser markings obtained in Formulations 1 and 2. To illustrate, faster laser movement speeds can be used and/or higher frequencies can be used to generate similar intensity marks with Formulation 3. Higher laser movement speeds and higher frequencies may generate less exposure to a substrate (e.g., substrate layer 122), and generally cause less damage or deformation in the substrate. Thus, a substrate having Formulation 3 can have laser marks inscribed with less exposure (e.g., more efficiently) and with less reduction in structural integrity.

Referring to FIG. 4, an example of a system 400 for producing a molded part is shown. System 400 is configured to use an extrusion process to form a polymer composition and an injection molding process to form a molded part (e.g., part 112 and/or substrate layer 122 thereof) from the polymer composition, as described herein. System 400 includes an extruder 410, an injector 414, and a die 418 (e.g., a mold). Extruder 410 is coupled to injector 414 via one or more conduits 422, such as one or more tubes. Injector 414 is coupled to die 418 via one or more conduits 426, such as one or more tubes. System 400 may be controlled by a controller 412, such as processor 164 and/or a forming controller thereof. For example, controller 412 sends one or more control signals 482 to initiate one or more of the operations of system 400.

Extruder 410 includes one more hoppers, such as a first hopper 430 and a second hopper 432, and a barrel 434 coupled to the one or more hoppers. For example, barrel 434 may be coupled to a hopper via a via a feed throat 438. Each hopper 430, 432 is configured to receive material (e.g., pellets, granules, flakes, powders, and/or liquids) that is provided (e.g., gravity fed or force fed) from the hopper to barrel 434 via a corresponding feed throat 438. As shown, first hopper 430 has received a first material 440 and second hopper 432 has received a second material 442. First material 440 includes a polymer, such as polymer 132, and second material 442 includes a laser marking additive 134. Although described as being provided to separate hoppers, in other implementations, first and second materials 440, 442 may be provided by the same hopper.

In some implementations, polymer 132 includes polycarbonate (PC), polycarbonate copolymer (PC COPO), polycarbonate-siloxane copolymers, polyetherimide, polyetherimide-siloxane copolymers, polymethylmethacrylate (PMMA), polyphenylene ether (PPE)-siloxane copolymers, polylactic acid, bisphenol-A based polycarbonate copolymers, or a combination thereof.

Additionally or alternatively, polymer 132 may include thermoplastic polymers, copolymers, and blends thereof. The polymer 132 may include a polyester, such as a semicrystalline polymer, polyethylene terephthalate (PET), polyethylene terephthalate glycol-modified (PETG), glycol-modified poly-cyclohexylenedimethylene terephthalate (PCTG), polycyclohexylenedimethylene terephthalate (PCT), isophthalic acid-modified polycyclohexylenedimethylene terephthalate (PCTA), and Tritan^{™} (a combination of dimethyl terephthalate, 1,4-cyclohexanedimethanol, and 2,2,4,4-tetramethyl-1,3-cyclobutanediol from Eastman Chemical). Additionally, or alternatively, the material may include a resin, such as Xylex^{™} (a combination of PC and an amorphous polyester), polybutylene terephthalate (PBT) (e.g., a resin from the Valox^{™} line of PBT), and/or a PET resin available from SABIC^{™}. Additionally, or alternatively, the material may include liquid-crystal polymer (LCP), polyether ether ketone (PEEK), fluorinated ethylene propylene (FEP), polysulfone (PSU), polyethylenimine, polyimide (PI), cyclic olefin copolymer (COC), cyclo olefin polymer (COP), polyamide (PA), acrylonitrile butadiene styrene (ABS), or a combination thereof.

The term "polycarbonate" (or "polycarbonates"), as used herein, includes copolycarbonates, homopolycarbonates and (co)polyester carbonates. The term polycarbonate can be further defined as compositions have repeating structural units of the formula (1): 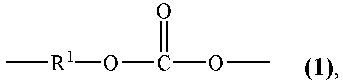 in which at least 60 percent of the total number of R1 groups are aromatic organic radicals and the balance thereof are aliphatic, alicyclic, or aromatic radicals. In a further aspect, each R1 is an aromatic organic radical and, more preferably, a radical of the formula (2):

HO-A¹-Y¹-A²-OH (2),

where each of A1 and A2 is a monocyclic divalent aryl radical and Y1 is a bridging radical having one or two atoms that separate A1 from A2. In various aspects, one atom separates A1 from A2. For example, radicals of this type include, but are not limited to, radicals such as -O-, -S-, -S(O) -, -S(O2) -, -C(O) -, methylene, cyclohexyl-methylene, 2-[2.2.1]-bicycloheptylidene, ethylidene, isopropylidene, neopentylidene, cyclohexylidene, cyclopentadecylidene, cyclododecylidene, and adamantylidene. The bridging radical Y1 may be a hydrocarbon group or a saturated hydrocarbon group, such as methylene, cyclohexylidene, or isopropylidene, as illustrative, non-limiting examples.

In some implementations, another material can be combined with first and second materials 440, 442 in the extruder 410. For example, the other material may be received by the extruder 410 via the one or more hoppers. The other material can include one or more additives, such as an impact modifier, flow modifier, antioxidant, heat stabilizer, light stabilizer, ultraviolet (UV) light stabilizer, UV absorbing additive, plasticizer, lubricant, antistatic agent, antimicrobial agent, colorant (e.g., a dye or pigment), surface effect additive, radiation stabilizer, or a combination thereof, as illustrative, non-limiting examples.

Exemplary UV absorbing additives include hydroxybenzophenones; hydroxybenzotriazoles; hydroxybenzotriazines; cyanoacrylates; oxanilides; benzoxazinones benzylidene malonates; hindered amine light stabilizers; nano-scale inorganics such as nickel quenchers, metal oxides, mixed metal oxides, metal borides; and combinations comprising at least one of the foregoing; 2- (2H-benzotriazol-2-yl)-4-(1,1,3,3-tetramethylbutyl)-phenol (CYASORB^{®} 5411); 2-hydroxy-4-n-octyloxybenzophenone (CYASORB^{®} 531); 2-[4,6-bis(2,4-dimethylphenyl)-1,3,5-triazin-2-yl]- 5-(octyloxy)-phenol (CYASORB^{®} 1164); 2,2'-(1,4- phenylene)bis(4H-3,1-benzoxazin-4-one) (CYASORB^{®} UV-3638); 1,3-bis[(2-cyano-3,3-diphenylacryloyl)oxy]-2,2-bis[[(2-cyano-3, 3-diphenylacryloyl)oxy]methyl]propane (UVINUL^{®} 3030); 2,2'-(1,4-phenylene) bis(4H-3,1-benzoxazin-4-one); 1,3-bis[(2-cyano-3,3-diphenylacryloyl)oxy] -2,2-bis[[(2-cyano-3,3-diphenylacryloyl)oxy]methyl]propane; nano-size inorganic materials such as titanium oxide, cerium oxide, and zinc oxide, all with particle size less than or equal to 100 nanometers, or combinations comprising at least one of the foregoing UV absorbers. UV absorbers are used in amounts of 0.001 to 5 parts by weight, based on 100 parts by weight of the total composition, excluding any filler. Inorganic additives such as lanthanum hexaboride (LaB6) or Cesium tungsten oxide (CTO) can also be used to enhance the interaction of compositions with laser beams and improve mark contrasts.

Colorants such as pigment and/or dye additives can also be present alone or in combination with UV absorbing stabilizers having little residual visible coloration in order to modulate the substrate color as well as the color and contrast of the laser inscribed text, logos, barcodes, images, etc. by directly contributing to the change of reflectivity. Useful pigments can include, organic pigments such as azos, di-azos, quinacridones, perylenes, naphthalene tetracarboxylic acids, flavanthrones, isoindolinones, tetrachloroisoindolinones, anthraquinones, enthrones, dioxazines, phthalocyanines, and azo lakes; Pigment Red 101, Pigment Red 122, Pigment Red 149, Pigment Red 177, Pigment Red 179, Pigment Red 202, Pigment Violet 29, Pigment Blue 15, Pigment Blue 60, Pigment Green 7, Pigment Yellow 119, Pigment Yellow 147, Pigment Yellow 150, and Pigment Brown 24; or combinations comprising at least one of the foregoing pigments.

Exemplary dyes are generally organic materials and include coumarin dyes such as coumarin 460 (blue), coumarin 6 (green), nile red or the like; lanthanide complexes; hydrocarbon and substituted hydrocarbon dyes; polycyclic aromatic hydrocarbon dyes; scintillation dyes such as oxazole or oxadiazole dyes; aryl- or heteroaryl-substituted poly (C2-8) olefin dyes; carbocyanine dyes; indanthrone dyes; phthalocyanine dyes; oxazine dyes; carbostyryl dyes; napthalenetetracarboxylic acid dyes; porphyrin dyes; bis(styryl)biphenyl dyes; acridine dyes; anthraquinone dyes; cyanine dyes; methine dyes; arylmethane dyes; azo dyes; indigoid dyes, thioindigoid dyes, diazonium dyes; nitro dyes; quinone imine dyes; aminoketone dyes; tetrazolium dyes; thiazole dyes; perylene dyes, perinone dyes; bis-benzoxazolylthiophene (BBOT); triarylmethane dyes; xanthene dyes; thioxanthene dyes; naphthalimide dyes; lactone dyes; fluorophores such as anti-stokes shift dyes which absorb in the near infrared wavelength and emit in the visible wavelength, or the like; luminescent dyes such as 7-amino-4-methylcoumarin; 3-(2'-benzothiazolyl)-7-diethylaminocoumarin; 2-(4-biphenylyl)-5-(4-t-butylphenyl)-1,3,4-oxadiazole; 2,5-bis-(4-biphenylyl)-oxazole; 2,2'-dimethyl-p-quaterphenyl; 2,2-dimethyl-p-terphenyl; 3,5,3"",5""-tetra-t-butyl-p-quinquephenyl; 2,5-diphenylfuran; 2,5-diphenyloxazole; 4,4'-diphenylstilbene; 4-dicyanomethylene-2-methyl-6-(p-dimethylaminostyryl)-4H-pyran; 1,1'-diethyl-2,2'-carbocyanine iodide; 3,3'-diethyl-4,4',5,5'-dibenzothiatricarbocyanine iodide; 7-dimethylamino-1-methyl-4-methoxy-8-azaquinolone-2; 7-dimethylamino-4-methylquinolone-2; 2-(4-(4-dimethylaminophenyl)-1,3-butadienyl)-3-ethylbenzothiazolium perchlorate; 3-diethylamino-7-diethyliminophenoxazonium perchlorate; 2-(1-naphthyl)-5-phenyloxazole; 2,2'-p-phenylen-bis(5-phenyloxazole); rhodamine 700; rhodamine 800; pyrene, chrysene, rubrene, coronene, or the like; or combinations comprising at least one of the foregoing dyes. It is to be understood that any of the above described additives, UV absorbers, colorants, etc. can be used with any of the materials described herein and is not limited to polycarbonate.

Each hopper 430, 432 provides its corresponding material 440, 442 into barrel 434 where the materials are combined to form a polymer composite 450. For example, the materials are gradually melted in barrel 434 by the mechanical energy (e.g., pressure) generated by turning screws, by heaters arranged along barrel 434, or both. The molten materials are mixed together (e.g., blended) to form polymer composite 450. Optionally, one or more additives can be mixed at a suitable time during the mixing of the components for forming the polymer composite 450.

Thus, polymer composite 450 has increased UV absorption, as compared to polymer 132, by incorporating the laser marking additive 134 described herein into polymer composite 450. The increased UV absorption enables laser marking in transparent or translucent parts or layers made using polymer composite 450. For example, parts made from polymer composite 450 may form white or light colored marks when exposed to UV radiation, such as a laser beam from a UV laser.

Polymer composite 450 is provided from barrel 434 via conduit 422 to injector 414. Injector 414 injects polymer composite 450 into die 418 via conduit 426. Polymer composite 450 flows into the die 418 until the polymer composite 450 substantially fills the die 418, such as one or more cavities or features thereof. The polymer composite 450 cools to form the molded part (e.g., part 112).

As shown, polymer composite 450 is provided from extruder 410 to die 418 via injector 414. In other implementations, system 400 may not include injector and extruder 410 may provide polymer composite 450 to die 418 via one or more conduits. Although polymer composite 450 has been described in system 400 using an extrusion process, in other implementations, polymer composite 450 may be formed by another process and provided to injector 414 for injection into die 418.

As described with reference to FIG. 4, system 400 is configured to form a molded part. The molded part includes a polymer composite (including the polymer and the laser marking additive) that may advantageously enable laser marking by UV radiation. For example, a substrate layer of the molded part forms white or light colored laser marking when exposed to a UV laser. Additionally, the substrate layer may be transparent or translucent.

In other implementations, the extruder 410 forms extrudate (e.g., strands of polymer composite 450) which is then cooled in a water bath, or by spraying the extrudate in a conduit 422 (e.g., a conveyor belt) as the extrudate moves from extruder 410 to a granulator via conduit 422. The granulator breaks the extrudate (e.g., the strands thereof) into pieces, such as pellets. The pellets of polymer composite 450 can then be used in an injection molding process or in another molding process.

In some implementations, the polymer composite 450 may further include one or more additives intended to impart certain characteristics to molded part (e.g., part 112). The various additives may be incorporated into polymer composite 450, with the proviso that the additive(s) are selected so as to not significantly adversely affect the desired properties of polymer composite 450 (e.g., the additives have good compatibility with the polymer 132 and the laser marking additive 134). For example, the additive(s) selected do not significantly adversely affect bonding between polymer 132 and laser marking additive 134, transparency of the polymer 132, UV absorption of the laser marking additive 134, or a combination thereof.

As an illustrative, non-limiting example, polymer composite 450 may include a mold release agent to facilitate ejection of a molded part from the mold assembly. Examples of mold release agents include both aliphatic and aromatic carboxylic acids and their alkyl esters, such as stearic acid, behenic acid, pentaerythritol tetrastearate, glycerin tristearate, and ethylene glycol distearate, as illustrative, non-limiting examples. Mold release agents can also include polyolefins, such as high-density polyethylene, linear low-density polyethylene, low-density polyethylene, and similar polyolefin homopolymers and copolymers. Additionally, some compositions of mold release agents may use pentaerythritol tetrastearate, glycerol monostearate, a wax, or a poly alpha olefin. Mold release agents are typically present in the composition at 0.05 to 10 wt%, based on total weight of the composition, such as 0.1 to 5 wt %, 0.1 to 1 wt%, or 0.1 to 0.5 wt%. Some mold release agents have high molecular weight, typically greater than or equal to 300, to prohibit loss of the release agent from the molten polymer mixture during melt processing.

Referring to FIG. 5, an example of a method of inscribing a substrate is shown. Method 500 may be performed by a laser device or a system, such as system 100 or one or more components thereof (e.g., laser device 114, laser 142, electronic device 116, laser controller 172, or a combination thereof). The substrate may include or correspond to part 112, a portion of part 112, such as substrate layer 122, or the molded part of FIG. 4, as described herein.

Method 500 includes initiating application of a laser beam to the substrate, at 510. For example, laser beam may include or correspond to laser beam 152 of FIG. 1. To illustrate, laser device 114 causes laser 142 to generate and provide laser beam 152 to substrate layer 122 responsive to control signal(s) from laser controller 172 of electronic device 116 and. Additionally, or alternatively, laser device 114 causes laser 142 to generate laser beam 152 responsive to user inputs received via one or more I/O devices 170 of laser device 114. Application of laser beam 152 may pass through one or more outer layers (e.g., 124) prior to reaching the substrate. In a particular implementation, the substrate is incorporated in a medical device before or after inscription.

Method 500 optionally includes initiating movement of a laser device such that the laser beam moves in a pattern that corresponds to a laser inscription, at 512. For example, laser inscription may include or correspond to laser indicia 156 and may include one or more laser markings 154. To illustrate, laser device 114 adjusts laser 142 to move or direct laser beam 152 responsive to control signal(s) from laser controller 172 of electronic device 116 or user inputs received via one or more I/O devices of laser device 114. Moving laser beam 152 may generate multiple laser markings 154 and form a pattern of laser markings 154, i.e., a laser indicia 156. In other implementations, the substrate or the substrate and the laser device are moved such that the laser beam moves with respect to the substrate in a pattern.

Method 500 further includes ceasing the application of the laser beam to the substrate, at 514. For example, laser device 114 causes laser 142 to cease or stop generation of laser beam 152 responsive to control signal(s) from laser controller 172 of electronic device 116 or user inputs received via one or more I/O devices 170 of laser device 114. One product or article of manufacture that can be formed from method 500 includes part 112.

Thus, method 500 describes manufacturing of a polymer composition, such as polymer composite 450. Method 500 advantageously enables creating a polymer composition with increased UV absorption such that laser markings can be formed in transparent or translucent materials, such as substrate layer 122, by a UV laser.

Referring to FIG. 6, an example of a method of manufacturing a polymer composition is shown. Method 600 may be performed by a manufacturing device or system, such as system 400 (e.g., extruder 410). The polymer composition may include or correspond to polymer composite 450, as described herein. Polymer composition may be used to form part 1122 or a portion thereof, such as substrate layer 122.

Method 600 includes receiving a laser marking additive, at 610. For example, the laser marking additive may include or correspond to laser marking additive 134, such as described with reference to FIG. 1. Method 600 also includes combining the laser marking additive with one or more polymers to form a polymer composition, at 612. One product or article of manufacture that can be formed from method 600 includes part 112. Optionally, method 600 includes receiving one or more polymers prior to combining the laser marking additive with the one or more polymers, such as polymer 132.

Thus, method 600 describes manufacturing of a polymer composition, such as polymer composite 450. Method 600 advantageously enables creating a polymer composition with increased UV absorption such that laser markings can be formed in transparent or translucent materials, such as substrate layer 122, by a UV laser.

Referring to FIG. 7, an example of a method of manufacturing a part is shown. Method 700 may be performed by a manufacturing device or system, such as system 400 (e.g., extruder 410). The part may include or correspond to part 112 or the molded part of FIG. 4, as described herein.

Method 700 includes injecting a polymer composition into a mold, at 710. The mold defines one or more cavities such that, when injected, the polymer composition flows into the mold until the polymer composition substantially fills the one or more cavities. For example, the polymer composition may include or correspond to polymer composite 450, and the mold may include or correspond to a mold or die 418. To illustrate, polymer composite 450 is in a molten state when applied to the die 418 and is cured to form the part 112 (e.g., substrate layer 122 thereof) by cooling in the die 418. Method 700 also includes removing the part from the mold, at 712. To illustrate, one or more pieces of die 418 are decoupled from one another and part 112 is removed from die 418.

Method 700 may further include combining, at an extrusion device, a polymer, a laser marking additive, and one or more additives to form the polymer composition. For example, the extrusion device may include or correspond to extruder 410. The one or more additives may include or correspond to additive 134, additive(s) described with reference to FIG. 4, or a combination thereof. In some such implementations, method 700 also includes providing, from the extrusion device to an injection device, the polymer composition for injection into the mold. For example, the injection device may include or correspond to injector 414. In other implementations, the extrusion device may inject the polymer composition into the mold.

Thus, method 700 describes manufacturing of a part, such as part 112, having UV absorbers. Method 700 advantageously enables forming parts capable of laser inscription with a UV laser. Additionally, the laser inscription may have increased wear and tamper resistance as compared to laser inscriptions formed with IR lasers.

It is noted that one or more operations described with reference to one of the methods of FIGS. 5-7 may be combined with one or more operations of another of FIGS. 5-7. For example, one or more operations of method 600 may be combined with one or more operations of method 700. Additionally, one or more of the operations described with reference to the systems of FIGS. 1 and 4 may be combined with one or more operations described with reference to one of the methods of FIGS. 5-7.

The above specification and examples provide a complete description of the structure and use of illustrative implementations. Although certain implementations have been described above with a certain degree of particularity, or with reference to one or more individual implementations, those skilled in the art could make numerous alterations to the disclosed implementations without departing from the scope of this disclosure. As such, the various illustrative implementations of the methods and systems are not intended to be limited to the particular forms disclosed. Rather, they include all modifications and alternatives falling within the scope of the claims, and implementations other than the one shown may include some or all of the features of the depicted implementations. For example, elements may be omitted or combined as a unitary structure, connections may be substituted, or both. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one implementation or may relate to several implementations. Accordingly, no single implementation described herein should be construed as limiting and implementations of the disclosure may be suitably combined without departing from the teachings of the disclosure.

The claims are not intended to include, and should not be interpreted to include, means-plus- or step-plus-function limitations, unless such a limitation is explicitly recited in a given claim using the phrase(s) "means for" or "step for," respectively.

## Claims

1. A composition comprising:
one or more polymers; and
a combined ultraviolet absorbing laser marking additive consisting of: 2-(4,6-Diphenyl-1,3,5-triazin-2-yl- 5-hexyloxy)phenol, 2-(2h-benzotriazol-2-yl)-4-(1,1,3,3-tetramethylbutyl)phenol, 2-(2-hydroxy-3,5 dicumyl)benzotriazole, and any combination thereof,
wherein a weight percentage concentration of 2-(4,6-Diphenyl-1,3,5-triazin-2-yl-5-hexyloxy)phenol is within a range of 0.01% to 0.5 % by weight of the composition,
wherein a weight percentage concentration of 2-(2h-benzotriazol-2-yl)-4-(1,1,3,3-tetramethylbutyl)phenol is within a range of 0.01% to 0.5 % by weight of the composition, and
wherein a weight percentage concentration of 2-(2-hydroxy-3,5 dicumyl)benzotriazole is within a range of 0.01% to 3% by weight of the composition.

2. The composition of claim 1, wherein at least one polymer of the one or more polymers is selected from the group consisting of: polycarbonate (PC), polymethyl methacrylate (PMMA), cyclic olefin copolymers (COCs), cyclic olefin polymers (COPs), cyclic block copolymers (CBC), pentaerythritol tetrastearate (PET), polyvinyl chloride (PVC), polyethylene (PE), polypropylene (PP), polystyrene (PS), polymetylpentene (PMP), isosorbide based polycarbonate, styrene acrylonitrile (SAN), acrylonitrile butadiene styrene (ABS), and any combination thereof.

3. The composition of claim 1, wherein at least one polymer of the one or more polymers is selected from the group consisting of: cyclic olefin copolymers (COCs), cyclic olefin polymers (COPs), cyclic block copolymers (CBC), polymetylpentene (PMP), polyolefins, and any combination thereof.

4. The composition of any of claims 1-3, wherein the one or more polymers include polycarbonate, and wherein 2-(2 hydroxy-3,5 dicumyl)benzotriazole is present in an amount less than or equal to 3 % by weight of the composition.

5. The composition of any of claims 1-6, wherein the one or more polymers include pentaerythritol tetrastearate (PET), and wherein 2-(2 hydroxy - 3,5 dicumyl)benzotriazole is present in an amount less than or equal to 0.5 % by weight of the composition.

6. A part including a substrate formed from the composition of any of claims 1-5, wherein the substrate is transparent or translucent, wherein the ultraviolet absorbing laser marking additive enables formation of the laser marking responsive to receiving UV light from a UV laser, and wherein the laser marking formed in the substrate by a UV laser is light-colored or white.

7. The part of claim 6, wherein the part is a medical device or is incorporated into a medical device.

8. A method of inscribing a substrate, the method comprising:
at the substrate comprising
one or more polymers and
two or more ultraviolet absorbing laser marking additive selected from the group consisting of: 2-(4,6-Diphenyl-1,3,5-triazin-2-yl- 5-hexyloxy)phenol, 2-(2h-benzotriazol-2-yl)-4-(1,1,3,3-tetramethylbutyl)phenol, 2-(2 hydroxy - 3,5 dicumyl)benzotriazole, and any combination thereof,
wherein a weight percentage concentration of 2-(4,6-Diphenyl-1,3,5-triazin-2-yl-5-hexyloxy)phenol is within a range of 0.01% to 0.5 % by weight of the composition,
wherein a weight percentage concentration of 2-(2h-benzotriazol-2-yl)-4-(1,1,3,3-tetramethylbutyl)phenol is within a range of 0.01% to 0.5 % by weight of the composition, and
wherein a weight percentage concentration of 2-(2-hydroxy-3,5 dicumyl)benzotriazole is within a range of 0.01% to 3% by weight of the composition;
performing,
initiating application of a laser beam to the substrate, wherein the laser beam has a wavelength of 500 nm or less; and
ceasing the application of the laser beam to the substrate, wherein the application of the laser beam generates a laser marking on the substrate,
wherein the ultraviolet absorbing laser marking additive enables formation of the laser marking.

9. The method of claim 8, further comprising initiating movement of a laser device such that the laser beam moves in a pattern that corresponds to a laser inscription.

10. The method of claim 9, wherein the laser beam has a wavelength of 400 nm or less, and wherein the laser beam passes through one or more layers prior to impinging on the substrate.

11. The method of claim 10, wherein a laser device that generates the laser beam has one or more operating characteristics selected from the group of operating characteristics consisting of: a power output between 1 and 3 Watts (W), a frequency between 10 and 50 kilohertz (kHz), a movement speed between 200 and 1600 millimeters per second (mm/s), and a power setting of 95%.

12. A part including a substrate having a laser marking formed by the method of any of claims 8-11.

## Patentansprüche

1. Eine Zusammensetzung umfassend:
ein oder mehrere Polymere; und
ein kombiniertes Ultraviolett absorbierendes Lasermarkierungsadditiv bestehend aus: 2-(4,6-Diphenyl-1,3,5-triazin-2-yl-5-hexyloxy)phenol, 2-(2h-Benzotriazol-2-yl)-4-(1,1,3,3-tetramethylbutyl)phenol, 2-(2-Hydroxy-3,5-dicumyl)benzotriazol und jede Kombination davon,
wobei eine gewichtsprozentuale Konzentration von 2-(4,6-Diphenyl-1,3,5-triazin- 2-yl-5-Hexyloxy)phenol in einem Bereich von 0,01 % bis 0,5 % des Gewichts der Zusammensetzung liegt,
wobei die gewichtsprozentuale Konzentration von 2-(2h-Benzotriazol-2-yl)-4-(1,1,3,3-Tetramethylbutyl)-phenol in einem Bereich von 0,01 bis 0,5 Gew.-% der Zusammensetzung liegt, und
wobei die gewichtsprozentuale Konzentration von 2-(2-Hydroxy-3,5-dicumyl)benzotriazol in einem Bereich von 0,01 bis 3 Gew.-% der Zusammensetzung liegt.

2. Zusammensetzung nach Anspruch 1, wobei mindestens ein Polymer des einen oder der mehreren Polymere ausgewählt ist aus der Gruppe bestehend aus: Polycarbonat (PC), Polymethylmethacrylat (PMMA), zyklische Olefin-Copolymere (COCs), zyklische Olefin-Polymere (COPs), zyklische Block- Copolymere (CBC), Pentaerythritol-Tetrastearat (PET), Polyvinylchlorid (PVC), Polyethylen (PE), Polypropylen (PP), Polystyrol (PS), Polymetylpenten (PMP), Polycarbonat auf Isosorbidbasis, Styrol-Acrylnitril (SAN), Acrylnitril-Butadien-Styrol (ABS) und jede Kombination davon.

3. Zusammensetzung nach Anspruch 1, wobei mindestens ein Polymer des einen oder der mehreren Polymere ausgewählt ist aus der Gruppe bestehend aus: cyclischen Olefin-Copolymeren (COCs), cyclischen Olefin-Polymeren (COPs), cyclischen BlockCopolymeren (CBC), Polymethylpenten (PMP), Polyolefinen und jede Kombination davon.

4. Zusammensetzung nach einem der Ansprüche 1 bis 3, wobei das eine oder die mehreren Polymere Polycarbonat einschließen und wobei 2-(2-Hydroxy-3,5-dicumyl)benzotriazol in einer Menge von weniger als oder gleich 3 Gew.-% der Zusammensetzung vorhanden ist.

5. Zusammensetzung nach einem der Ansprüche 1 bis 6, wobei das eine oder die mehreren Polymere Pentaerythrittetrastearat (PET) einschließen, und wobei 2-(2-Hydroxy-3,5-dicumyl)benzotriazol in einer Menge von weniger als oder gleich 0,5 Gew.-% der Zusammensetzung vorhanden ist.

6. Teil mit einem Substrat, das aus der Zusammensetzung nach einem der Ansprüche 1 bis 5 gebildet ist, wobei das Substrat transparent oder lichtdurchlässig ist, wobei das ultraviolettabsorbierende Lasermarkierungsadditiv die Bildung der Lasermarkierung als Reaktion auf den Empfang von UV-Licht von einem UV-Laser ermöglicht und wobei die in dem Substrat durch einen UV-Laser gebildete Lasermarkierung hell gefärbt oder weiß ist.

7. Das Teil nach Anspruch 6, wobei das Teil ein medizinisches Gerät ist oder in ein medizinisches Gerät eingebaut ist.

8. Verfahren zum Beschriften eines Substrats, wobei das Verfahren umfasst:
auf dem Substrat umfassend
ein oder mehrere Polymere und
zwei oder mehr ultraviolettabsorbierende Lasermarkierungsadditive, ausgewählt aus der Gruppe bestehend aus: 2-(4,6-Diphenyl-1,3,5-triazin-2-yl-5-hexyloxy)-phenol, 2-(2h-Benzotriazol-2-yl)-4-(1,1,3,3-tetramethylbutyl)- phenol, 2-(2-Hydroxy-3,5-dicumyl)-benzotriazol und jede Kombination davon,
wobei die gewichtsprozentuale Konzentration von 2-(4,6-Diphenyl-1,3,5-triazin-2-yl-5-hexyloxy)phenol in einem Bereich von 0,01 bis 0,5 Gew.-% der Zusammensetzung liegt,
wobei die gewichtsprozentuale Konzentration von 2-(2h-Benzotriazol-2-yl)-4-(1,1,3,3-Tetramethylbutyl)-phenol in einem Bereich von 0,01 bis 0,5 Gew.-% der Zusammensetzung liegt, und
worin die gewichtsprozentuale Konzentration von 2-(2-Hydroxy-3,5-dicumyl)benzotriazol in einem Bereich von 0,01 bis 3 Gew.-% der Zusammensetzung liegt;
Durchführung,
Initiieren der Anwendung eines Laserstrahls auf das Substrat, wobei der Laserstrahl eine Wellenlänge von 500 nm oder weniger hat; und
Beenden der Anwendung des Laserstrahls auf das Substrat, wobei die Einwirkung des Laserstrahls eine Lasermarkierung auf dem Substrat erzeugt,
wobei das ultraviolettabsorbierende Lasermarkierungsadditiv die Bildung der Lasermarkierung ermöglicht.

9. Das Verfahren nach Anspruch 8, ferner umfassend das Initiieren der Bewegung einer Laservorrichtung, so dass sich der Laserstrahl in einem Muster bewegt, das einer Laserbeschriftung entspricht.

10. Das Verfahren nach Anspruch 9, wobei der Laserstrahl eine Wellenlänge von 400 nm oder weniger hat und wobei der Laserstrahl eine oder mehrere Schichten durchläuft, bevor er auf das Substrat auftrifft.

11. Das Verfahren nach Anspruch 10, wobei eine Laservorrichtung, die den Laserstrahl erzeugt, eine oder mehrere Betriebscharakteristiken aufweist, die aus der Gruppe der Betriebscharakteristiken ausgewählt sind, die besteht aus: einer Ausgangsleistung zwischen 1 und 3 Watt (W), einer Frequenz zwischen 10 und 50 Kilohertz (kHz), einer Bewegungsgeschwindigkeit zwischen 200 und 1600 Millimetern pro Sekunde (mm/s) und einer Leistungseinstellung von 95%.

12. Teil mit einem Substrat, das eine Lasermarkierung aufweist, das durch das Verfahren nach einem der Ansprüche 8-11 gebildet wurde.

## Revendications

1. Une composition comprenant :
un ou plusieurs polymères ; et
un additif combiné de marquage laser absorbant les ultraviolets, composé de : 2-(4,6-diphényl-1,3,5-triazin-2-yl- 5-hexyloxy)phénol, 2-(2h-benzotriazol-2-yl)-4-(1,1,3,3-tétraméthylbutyl)phénol, 2-(2-hydroxy-3,5 dicumyl)benzotriazole, et toute combinaison de ceux-ci,
dans laquelle une concentration en pourcentage en poids de 2-(4,6-diphényl-1,3,5-triazin-2-yl-5-hexyloxy)phénol se situe dans une fourchette comprise entre 0,01 % et 0,5 % en poids de la composition,
dans laquelle une concentration en pourcentage en poids de 2-(2h-benzotriazol-2-yl)-4-(1,1,3,3-tétraméthylbutyl)phénol se situe dans une fourchette comprise entre 0,01 % et 0,5 % en poids de la composition, et
dans laquelle une concentration en pourcentage en poids de 2-(2-hydroxy-3,5 dicumyl)benzotriazole se situe dans une fourchette comprise entre 0,01 % et 3 % en poids de la composition.

2. La composition selon la revendication 1, dans laquelle au moins un polymère du ou des polymères est choisi dans le groupe constitué par : le polycarbonate (PC), le poly(méthacrylate de méthyle) (PMMA), les copolymères de cyclo oléfines (COCs), les polymères de cyclo oléfines (COPs), les copolymères à blocs cycliques (CBC), le tétrastéarate de pentaérythritol (PET), le chlorure de polyvinyle (PVC), le polyéthylène (PE), le polypropylène (PP), le polystyrène (PS), le polymétylpentène (PMP), le polycarbonate à base d'isosorbide, le styrène-acrylonitrile (SAN), l'acrylonitrile butadiène styrène (ABS), et toute combinaison de ceux-ci.

3. La composition selon la revendication 1, dans laquelle au moins un polymère du ou des polymères est choisi dans le groupe constitué par : les copolymères de cyclo oléfines (COCs), les polymères de cyclo oléfines (COPs), les copolymères à blocs cycliques (CBC), le polymétylpentène (PMP), les polyoléfines, et toute combinaison de ceux-ci.

4. La composition selon l'une quelconque des revendications 1 à 3, dans laquelle le ou les polymères comprennent le polycarbonate, et dans laquelle le 2-(2 hydroxy-3,5 dicumyl)benzotriazole est présent en une quantité inférieure ou égale à 3 % en poids de la composition.

5. La composition selon l'une quelconque des revendications 1 à 6, dans laquelle le ou les polymères comprennent le tétrastéarate de pentaérythritol (PET), et dans laquelle le 2-(2 hydroxy-3,5 dicumyl)benzotriazole est présent en une quantité inférieure ou égale à 0,5 % en poids de la composition.

6. Une pièce comprenant un substrat formé à partir de la composition selon l'une quelconque des revendications 1 à 5, dans laquelle le substrat est transparent ou translucide, l'additif de marquage laser absorbant les ultraviolets permettant la formation du marquage laser en réponse à la réception de lumière UV d'un laser UV, et le marquage laser formé dans le substrat par un laser UV étant de couleur claire ou blanc.

7. La pièce selon la revendication 6, dans laquelle la pièce est un dispositif médical ou est incorporée dans un dispositif médical.

8. Procédé d'inscription d'un substrat, comprenant :
au niveau du substrat comprenant
un ou plusieurs polymères et
deux ou plusieurs additifs de marquage laser absorbant les ultraviolets choisis dans le groupe constitué par : 2-(4,6-diphényl-1,3,5-triazin-2-yl- 5-hexyloxy)phénol, 2-(2h-benzotriazol-2-yl)-4-(1,1,3,3-tétraméthylbutyl)phénol, 2-(2 hydroxy-3,5 dicumyl)benzotriazole, et toute combinaison de ceux-ci,
dans lequel une concentration en pourcentage en poids de 2-(4,6-diphényl-1,3,5-triazin-2-yl-5-hexyloxy)phénol se situe dans une fourchette comprise entre 0,01 % et 0,5 % en poids de la composition,
dans lequel une concentration en pourcentage en poids de 2-(2h-benzotriazol-2-yl)-4-(1,1,3,3-tétraméthylbutyl)phénol se situe dans une fourchette comprise entre 0,01 % et 0,5 % en poids de la composition, et
dans lequel une concentration en pourcentage en poids de 2-(2-hydroxy-3,5 dicumyl)benzotriazole se situe dans une fourchette comprise entre 0,01 % et 3 % en poids de la composition ;
effectuer,
initier l'application d'un faisceau laser au substrat, le faisceau laser ayant une longueur d'onde de 500 nm ou moins ; et
cesser l'application du faisceau laser au substrat, l'application du faisceau laser générant un marquage laser sur le substrat,
dans lequel l'additif de marquage laser absorbant les rayons ultraviolets permet la formation du marquage laser.

9. Le procédé selon la revendication 8, comprenant en outre l'initiation d'un mouvement d'un dispositif laser de sorte que le faisceau laser se déplace selon un modèle qui correspond à une inscription laser.

10. Le procédé selon la revendication 9, dans lequel le faisceau laser a une longueur d'onde de 400 nm ou moins, et dans lequel le faisceau laser traverse une ou plusieurs couches avant d'impacter le substrat.

11. Le procédé selon la revendication 10, dans lequel un dispositif laser qui génère le faisceau laser a une ou plusieurs caractéristiques de fonctionnement choisies dans le groupe des caractéristiques de fonctionnement composé de : une puissance de sortie entre 1 et 3 Watts (W), une fréquence entre 10 et 50 kilohertz (kHz), une vitesse de mouvement entre 200 et 1 600 millimètres par seconde (mm/s), et un réglage de la puissance de 95%.

12. Pièce comprenant un substrat avec un marquage laser formé par le procédé selon l'une quelconque des revendications 8 à 11.
